(19) Europäisches Patentamt
European Patent Office
Office européen des brevets

(11) EP 4 692 049 A1

(12) EUROPEAN PATENT APPLICATION
published in accordance with Art. 153(4) EPC

(43) Date of publication:
11.02.2026 Bulletin 2026/07

(21) Application number: 24780939.5

(22) Date of filing: 01.04.2024

(51) International Patent Classification (IPC):
*C07C 233/36* *(2006.01)* *C07C 233/78* *(2006.01)*

(52) Cooperative Patent Classification (CPC):
C07C 233/36; C07C 233/78

(86) International application number:
PCT/JP2024/013474

(87) International publication number:
WO 2024/204847 (03.10.2024 Gazette 2024/40)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 30.03.2023 JP 2023055870

(71) Applicant: Mitsui Chemicals, Inc.
Tokyo 104-0028 (JP)

(72) Inventors:
- ISOGAI Makoto
Sodegaura-shi, Chiba 299-0265 (JP)
- TAKANO Shotaro
Sodegaura-shi, Chiba 299-0265 (JP)
- YANO Takaaki
Sodegaura-shi, Chiba 299-0265 (JP)
- KIMURA Takashi
Kuga-gun, Yamaguchi 740-0061 (JP)
- TAKEUCHI Takumi
Sodegaura-shi, Chiba 299-0265 (JP)

(74) Representative: J A Kemp LLP
80 Turnmill Street
London EC1M 5QU (GB)

# (54) AMIDE COMPOUND

(57) An amide compound having a structure of the following formula (1).

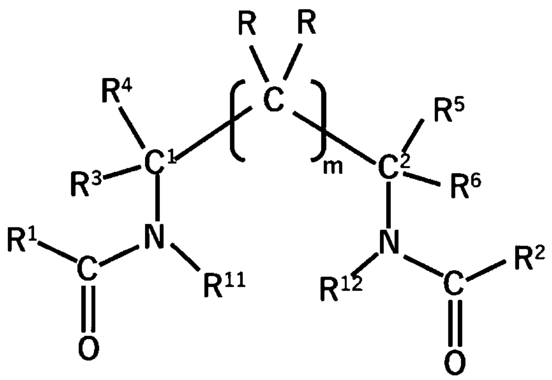

(1)

wherein C, $C^1$, and $C^2$ are carbon atoms, N is a nitrogen atom, $R^1$ and $R^2$ are each a hydrocarbon group, $R^{11}$ and $R^{12}$ are each a hydrogen atom or a hydrocarbon group, at least one group thereof is a hydrocarbon group, $R^3$ to $R^6$ are each a hydrogen atom or a hydrocarbon group, R is a hydrogen atom, and m is an integer of 1 to 5.

EP 4 692 049 A1

## Description

Technical Field

The present invention relates to a novel amide compound.

Background Art

Amide compounds are known to be in use in applications of not only intermediates of pharmaceuticals, such as solvents, medicines, and pesticides, but also, for example, nylon raw materials. It has been also reported that amide compounds are used as Mg compound-supported titanium catalysts that are used in olefin polymerization.

A catalyst for olefin polymerization is one of techniques greatly developed up to the present, which was triggered by the discovery of so-called Ziegler-Natta catalysts, for which Ziegler reported in 1953 that ethylene was polymerized even at low pressures by use of a combination of titanium tetrachloride and an organoaluminum compound and Natta subsequently reported the first propylene polymerization by use of a combination of titanium trichloride and a halogen-containing organoaluminum compound. Meanwhile, it has been found that catalysts containing titanium tetrachloride, a magnesium compound, and a Lewis base, which are referred to as third generation catalysts, can achieve both high polymerization activity (high productivity) and high stereoregularity in propylene polymerization. This provided one opportunity to allow propylene polymers (polypropylene) to spread around the world.

A Lewis base (hereinafter, also referred to as an "internal donor"), one of major components of the above third generation catalyst component (hereinafter, also referred to as a "solid titanium catalyst component"), was found to greatly affect catalyst performance, and various Lewis bases have been developed so far.

As Lewis bases for use in Ziegler-Natta catalysts, ethyl benzoate, phthalic esters, 1,3-diketone (Patent Literature 1), malonic ester (Patent Literature 2), succinic ester (Patent Literature 3), 2,4-pentanediol diester (Patent Literature 4), naphthalenediol diester (Patent Literature 5), and catechol diester (Patent Literature 6), for example, have been reported. Mainly enterprises vigorously make research and development in this field even today. It has been also reported that a diamide compound having a specific structure is suitable (Patent Literature 7, Patent Literature 8).

Citation List

Patent Literature

Patent Literature 1: JP2005-226076A
Patent Literature 2: JP2000-516987A
Patent Literature 3: JP2002-542347A
Patent Literature 4: JP2005-517746A
Patent Literature 5: JP2011-529888A
Patent Literature 6: JP2014-500390A
Patent Literature 7: Chinese Patent Publication No. 108570120
Patent Literature 8: Chinese Patent Publication No. 108570119


Summary of Invention

Technical Problem

Propylene polymers, while having heat resistance and rigidity similar to those of general-purpose engineering plastics, have an advantage of generating a smaller amount of toxic gas even when combustion-treated, because of being constituted substantially only by carbon and hydrogen.

With recent advances in molding techniques, use of a propylene polymer having higher stereoregularity than before has a possibility of developing higher physical properties (such as rigidity and heat resistance). For this reason, the market has required propylene polymers having higher stereoregularity. From the viewpoint of resource saving and environmental protection, methods for producing a propylene polymer at a high productivity also have been required.

It is thus an object of the present invention to provide an internal donor compound suitable for a solid titanium catalyst component capable of producing a propylene polymer having extremely high stereoregularity (that can be expected to have a high melting point or high heat of fusion) at a high productivity (high activity), when used mainly for solid titanium catalyst component.

Solution to Problem

As a result of diligent study to solve the above problems, the present inventors have found that an amide compound having a specific structure is suitable as, for example, a Lewis base for a solid titanium catalyst component, and completed the present invention. Examples of the present invention are shown below.

[1] An amide compound having a structure of the following formula (1):

[Chem. 1]

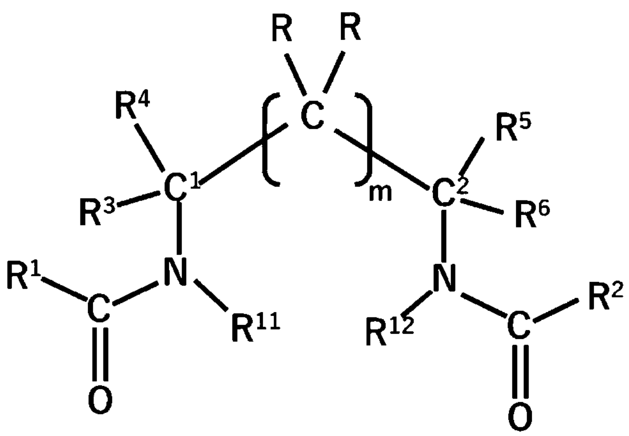

(1)

wherein

C, $C^1$, and $C^2$ are carbon atoms,
N is a nitrogen atom,
$R^1$ and $R^2$ are each a hydrocarbon group,
$R^{11}$ and $R^{12}$ are each a hydrogen atom or a hydrocarbon group, and at least one group thereof is a hydrocarbon group,
$R^3$ to $R^6$ are each a hydrogen atom or a hydrocarbon group,
R is a hydrogen atom, and
m is an integer of 1 to 5.

[2] The amide compound according to [1], wherein both the $R^{11}$ and $R^{12}$ are hydrocarbon groups.
[3] The amide compound according to [1] or [2], wherein the $R^1$, $R^2$, $R^4$, and $R^5$ are hydrocarbon groups composed of carbon and hydrogen.

Advantageous Effects of Invention

The amide compound of the present invention can be used as, for example, not only a raw material of a solvent, a pharmaceutical intermediate, or nylon but also a chelating agent or a Ziegler-Natta catalyst raw material.

Description of Embodiments

Hereinafter, an amide compound according to the present invention will now be described in detail.

[Amide compound]

The amide compound according to the present invention (hereinafter, also referred to as the "amide compound (A)") is represented by the following general formula (1):

[Chem. 2]

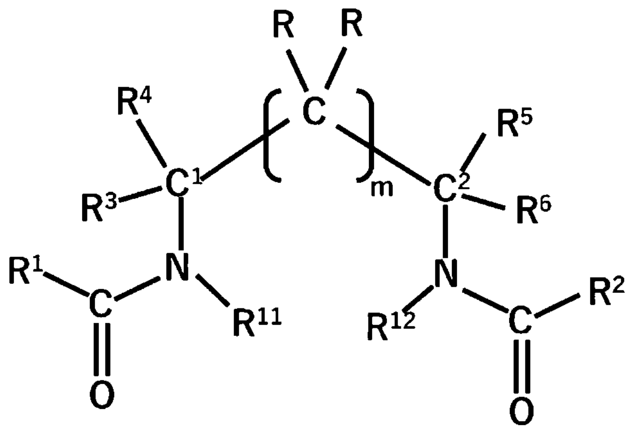

(1)

In the formula (1), C, $C^1$, and $C^2$ are carbon atoms, and N is a nitrogen atom. A line such as "-" indicates a covalent bond.

The $R^1$ and $R^2$ are each a hydrocarbon group. In more detail, the $R^1$ and $R^2$ are each a substituted or unsubstituted hydrocarbon group having 1 to 20 carbon atoms. Examples of the hydrocarbon group can include not only aliphatic hydrocarbon groups and alicyclic hydrocarbon groups but also substituted or unsubstituted hydrocarbon groups having an aryl group and 6 to 20 carbon atoms. Such a substituent may be a structure containing a heteroatom as described below. Examples of the heteroatom-containing aryl group can include those with a basic skeleton having a structure in which the aryl structure itself contains a heteroatom, such as a pyrrole ring or a pyran ring, and those in which a substituent such as a heteroatom-containing hydrocarbon group, for example, an alkoxy group, is bonded to a benzene ring.

The $R^{11}$ and $R^{12}$ are hydrogen atoms or hydrocarbon groups, and at least one thereof is a hydrocarbon group. Specific examples of the hydrocarbon groups can include the same substituents as the substituents exemplified as the $R^1$ and $R^2$. The $R^{11}$ and $R^{12}$ are both preferably hydrocarbon groups.

H in an amide compound having a "N-H" type structure as described above is so-called active hydrogen and is expected to be highly reactive with, for example, titanium in a solid titanium catalyst component for olefin polymerization, and it is thus considered that H is highly likely to be inappropriate as an electron donor component. However, the amide compound of the present invention exhibits an unexpected property of being suitable as the electron donor component in some cases.

$R^1$ and $R^2$ in the $R^1$ to $R^{12}$ are preferably substituted or unsubstituted hydrocarbon groups having an aryl group and 6 to 20 carbon atoms.

A representative example of the heteroatom-containing structure is a structure having a heteroatom-containing substituent, a preferable example of such a substituent is a heteroatom-containing aryl group, and a particularly preferable example is an oxygen-containing aryl group.

The $R^1$ and $R^2$ are preferably structures in which one carbon contained therein is covalently bonded to adjacent carbonyl carbon.

The $R^3$, $R^4$, $R^5$, and $R^6$ are each a hydrogen atom or a hydrocarbon group. The hydrocarbon group is a group including an aspect of having a heteroatom as described above. The heteroatom is preferably a group containing an element selected from Groups 15, 16, and 17 elements in the periodic table. More specific examples of $R^3$, $R^4$, $R^5$, and $R^6$ can include groups selected from a hydrogen atom, substituted or unsubstituted hydrocarbon groups having 1 to 20 carbon atoms, or halogen atoms.

The hydrocarbon group will be described in more detail. The hydrocarbon group of the present invention is a substituent essentially containing a carbon atom and a hydrogen atom and may be partially substituted with an atom selected from the group consisting of Groups 15 to 17 elements in the periodic table, such as a nitrogen atom, an oxygen atom, a phosphorus atom, and a halogen atom. The heteroatom may be substituted at one or multiple positions.

In the present invention, the term "atom" as in, for example, a halogen atom or a hydrogen atom in the description of a substituent may refer to, as a matter of course, aspects with a bond such as "H-" or "Cl-" when expressed in a structural formula.

The R is a hydrogen atom. In the present invention, a structure in which R's bonded to carbon adjacent to "$-(CR_2)_m-$" in the formula (1) are bonded to each other to form a double bond is included.

The "-$(CR_2)_m$-" structure is preferably a methylene chain structure.

The $R^1$, $R^2$, $R^3$, $R^4$, $R^5$, $R^6$, $R^{11}$, and $R^{12}$ can be bonded to each other to form a ring structure. Preferable aspects thereof will be described.

The $R^1$ and $R^2$ may be bonded to each other to form a ring structure. Substituents selected from $R^1$, $R^3$, $R^4$, and $R^{11}$ may be bonded to each other to form a ring structure. Substituents selected from $R^2$, $R^5$, $R^6$, and $R^{12}$ may be bonded to each other to form a ring structure.

A substituent selected from the substituents of the $R^3$ and $R^4$ and a substituent selected from the substituents of $R^5$ and $R^6$ may also be bonded to each other to form a ring structure.

A ring structure formed of substituents selected from the $R^3$ to $R^6$, being bonded to each other is preferably an alicyclic structure such as a five or more-membered ring, and more preferably a six or more-membered ring when mobility in the periphery of $C^1$ carbon or $C^2$ carbon in the formula (1) as described below is taken into account. Incidentally, the upper limit of the number of members in the ring structure is arbitrary, but a ten-membered ring is preferable, and an eight-membered ring is more preferable.

In a case where the $R^3$ and $R^4$ or the $R^5$ and $R^6$ form a ring structure, aspects in which a carbon-carbon double bond is formed are also included (at this time, the carbon-carbon double bond is regarded as a two-membered ring). On the other hand, in a case where $R^3$ to $R^6$ are bonded to each other to form a ring structure, this ring structure is preferably an alicyclic structure. The reason for such a structure being suitable for the present invention will be described below.

The $R^3$ to $R^6$ are preferably substituents of a relatively low bulk. In a solid titanium catalyst component containing an organic compound (that is generally called an internal donor in some cases), there is a tendency that there are many examples in which a highly bulky compound is suitable. The reason for the tendency of such a substituent of a low bulk being suitable being exhibited in the present invention is not clear at the moment, but it is considered to be because, probably as described below, the amide compound of the present invention tends to easily and stably obtain a relatively suitable conformation as, for example, a component of an olefin polymerization catalyst, and conversely, disturbance is thus less likely to occur in the coordination of an olefin to titanium in the solid titanium catalyst component when $R^3$ to $R^6$, which are substituents present on a side opposite to an amide group, are structures of a small bulk. $R^3$ and $R^6$ are more preferably hydrogen atoms.

The $R^{11}$ and $R^{12}$ are hydrocarbon groups, and an aspect in which these substituents are bonded to each other to form a ring structure is preferable in some cases. While the structure of the "-$(CR_2)_m$-" part in the formula (1) exhibits a relatively flexible structure, such a structure is considered to suppress the rotation of the amide group, have an effect of making it easy to obtain a conformation in which a motion, for example, a relatively large twist, occurs, thus, have an appropriate interaction with titanium in the solid titanium catalyst component, and probably, easily form a highly stereoregular active point.

m in the amide compound of the present invention is an integer of 1 to 5. A preferable lower limit value is 2 while also depending on the structure of the A. On the other hand, a preferable upper limit value is 4. Within such a range, the distance between at least two amide groups is in an appropriate range, and a suitable olefin polymerization performance can be expected to be exhibited.

The hydrocarbon groups that are contained in the $R^1$ to $R^{12}$ are monovalent hydrocarbon groups having 1 to 20 carbon atoms, preferably 1 to 10 carbon atoms, more preferably 2 to 8 carbon atoms, even more preferably 3 to 8 carbon atoms, yet more preferably 4 to 8 carbon atoms, and particularly preferably 4 to 6 carbon atoms. Examples of such hydrocarbon groups include aliphatic hydrocarbon groups, alicyclic hydrocarbon groups, and aromatic hydrocarbon groups, such as a methyl group, an ethyl group, a n-propyl group, an isopropyl group, a n-butyl group, an isobutyl group, a hexyl group, a heptyl group, an octyl group, a 2-ethylhexyl group, a decyl group, a dodecyl group, a tetradecyl group, a hexadecyl group, an octadecyl group, an eicosyl group, a cyclohexyl group, a substituted or unsubstituted aryl group such as a phenyl group, and a substituted or unsubstituted cycloalkenyl group. The alicyclic hydrocarbon groups and the aromatic hydrocarbon groups may contain a substituent. Among such substituents, preferable are, for example, a n-butyl group, an isobutyl group, a hexyl group, an octyl group, and a phenyl group, and more preferable are a n-butyl group, an isobutyl group, and a phenyl group.

In the case of the hydrocarbon groups containing Groups 15 to 17 elements in the periodic table, such as nitrogen, oxygen, and halogens, specifically, suitable examples thereof can include a carbonyl structure-containing group such as a carboxylic acid ester group, an aldehyde group, an acetyl group, or an oxycarbonylalkyl group, an alkoxy group, a substituted or unsubstituted alkoxy group, a substituted or unsubstituted alkenyloxy group, a substituted or unsubstituted cycloalkyloxy group, a substituted or unsubstituted cycloalkenyloxy group, a substituted or unsubstituted aryloxy group, a substituted or unsubstituted heteroaryl group, a substituted or unsubstituted heteroaryloxy group, and a siloxy group. The heteroatom is preferably nitrogen and oxygen, and more preferably oxygen.

The heteroatom-containing substituent is preferably an aryl group containing an oxygen-containing substituent, and a preferable example is, specifically, a structure in which an oxygen-containing substituent such as an alkoxy group, an aryloxy group, an alkoxyalkyl group, an aryloxyalkyl group, and a substituent in which oxygen of the above substituent is replaced with a carbonyl group or a carboxyl group is bonded to an aromatic skeleton. Among such substituents, a

substituent in which an alkoxy group or an aryloxy group is bonded to an aromatic skeleton is preferable, and a substituent in which an alkoxy group is bonded to an aromatic skeleton is more preferable. The number of carbon atoms in the oxygen-containing substituent is preferably 1 to 10, more preferably 1 to 8, and even more preferably 1 to 6. More specifically, preferably examples in addition to the methoxyphenyl group include an ethoxyphenyl group, a propyloxyphenyl group, an isopropyloxyphenyl group, a butoxyphenyl group, and a phenoxyphenyl group.

In the present invention, $R^1$, $R^2$, $R^3$, $R^4$, and $R^5$ are preferably hydrocarbon groups and more preferably hydrocarbon groups composed of carbon and hydrogen. In the present invention, the hydrocarbon group composed of carbon and hydrogen refers to a hydrocarbon group substantially containing no heteroatoms and consisting of carbon and hydrogen. In such a hydrocarbon group, the $R^1$ and $R^2$ are preferably substituents selected from alkyl groups and aromatic hydrocarbon groups. Meanwhile, $R^4$ and $R^5$ are preferably aliphatic hydrocarbon groups and more preferably alkyl groups.

In addition, the $R^1$, $R^2$, $R^3$, $R^4$, $R^5$, $R^6$, $R^{11}$, and $R^{12}$ are preferably independent groups (substituents) that are not bonded to each other in some cases.

In a case where one of the $R^{11}$ and $R^{12}$ is a hydrogen atom, the other substituent is preferably a hydrocarbon group including an aromatic structure. More specific examples thereof can include aliphatic hydrocarbon groups, substituents having a structure in which one hydrogen atom in an alicyclic hydrocarbon group is substituted into an aryl group, or aromatic hydrocarbon groups. Examples of such a substituent can include substituents having a structure in which some of aliphatic hydrocarbon groups, such as benzyl groups, 2-phenylethyl groups, 3-phenylpropyl groups, 2-phenylpropyl groups, or 4-phenylbutyl groups, have been substituted with an aryl group, such as a phenyl group. Examples thereof can include hydrocarbon groups having an aromatic structure and an alicyclic structure, such as a (phenylcyclohexyl)methyl group, a 2-(phenylcyclohexyl)ethyl group, and a 3-(phenylcyclohexyl)propyl group. Examples of the aromatic hydrocarbon groups can include a phenyl group, a naphthalyl group, an indenyl group, a fluorenyl group, and substituents having a structure obtained by adding hydrogen to the aromatic structure moiety of a part thereof.

Examples of such an amide compound include the following structures. Some structural formulae of the following exemplary compounds have stereoisomers, and even isomeric structures are clearly depicted for some exemplary compounds, but there may be isomeric structures that are not exemplified.

[Chem. 3]

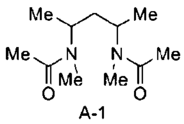
A-1

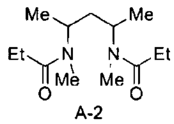
A-2

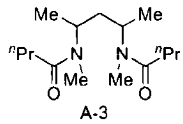
A-3

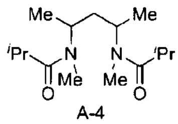
A-4

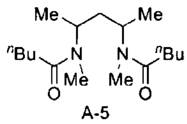
A-5

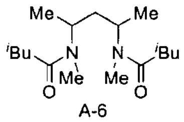
A-6

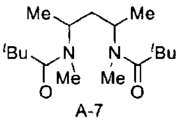
A-7

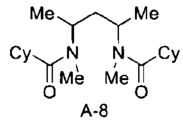
A-8

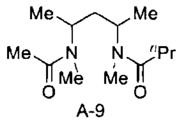
A-9

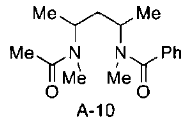
A-10

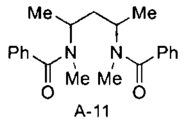
A-11

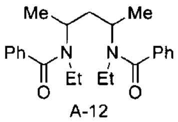
A-12

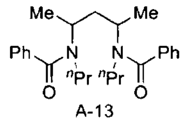
A-13

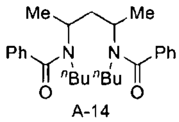
A-14

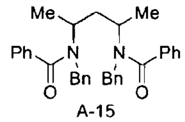
A-15

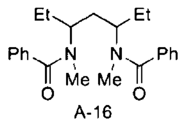
A-16

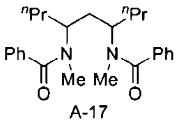
A-17

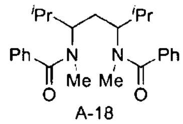
A-18

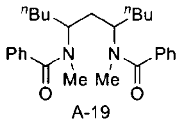
A-19

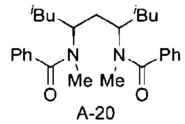
A-20

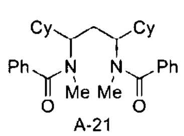
A-21

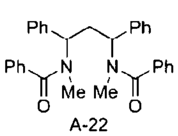
A-22

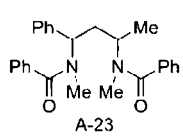
A-23

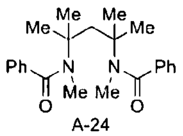
A-24

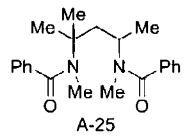
A-25

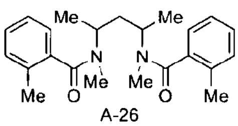
A-26

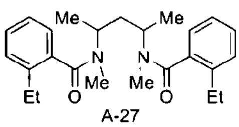
A-27

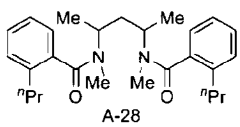
A-28

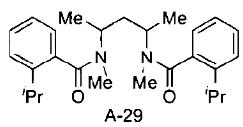
A-29

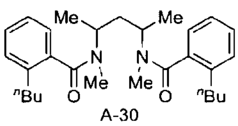
A-30

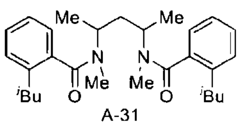
A-31

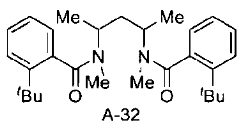
A-32

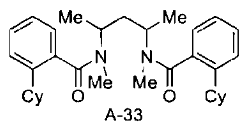
A-33

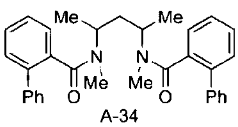
A-34

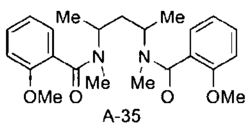
A-35

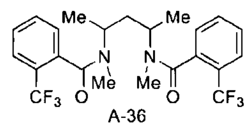
A-36

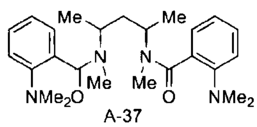
A-37

[Chem. 4]

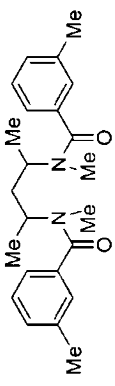
A-38

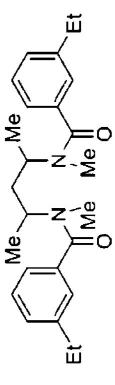
A-39

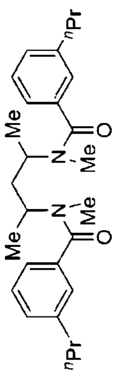
A-40

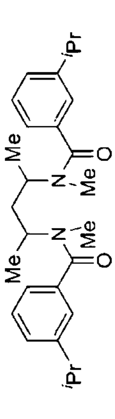
A-41

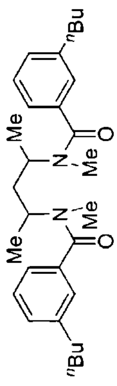
A-42

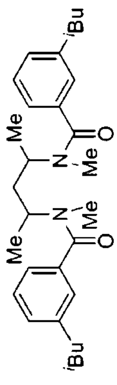
A-43

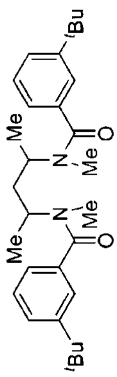
A-44

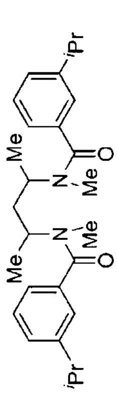
A-45

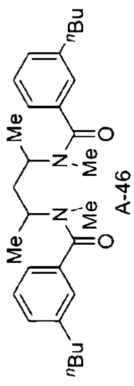
A-46

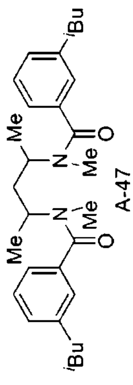
A-47

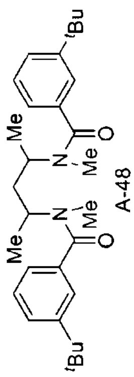
A-48

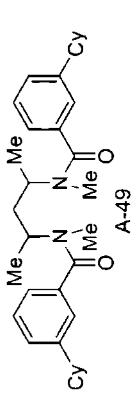
A-49

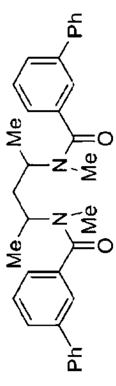
A-50

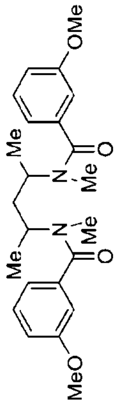
A-51

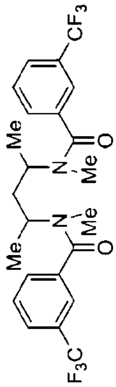
A-52

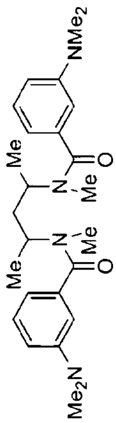
A-53

[Chem. 5]

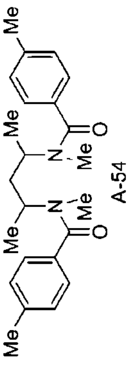
A-54

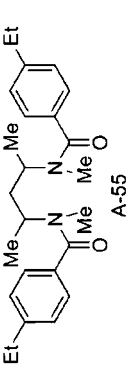
A-55

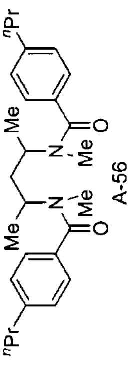
A-56

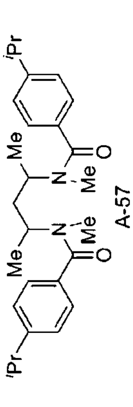
A-57

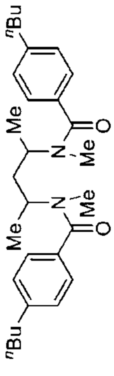
A-58

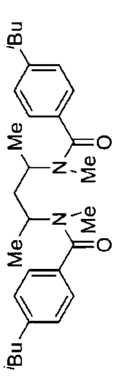
A-59

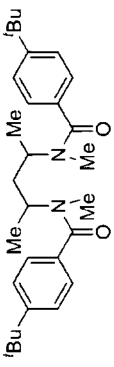
A-60

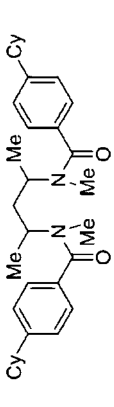
A-61

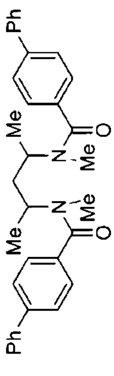
A-62

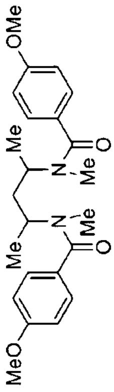
A-63

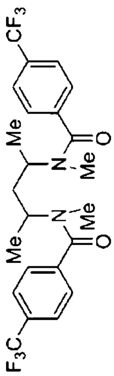
A-64

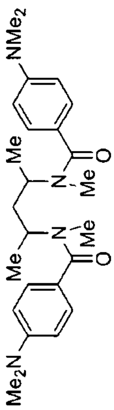
A-65

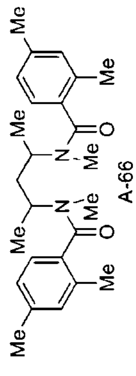
A-66

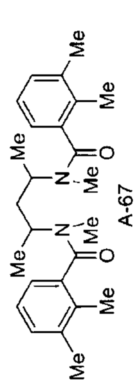
A-67

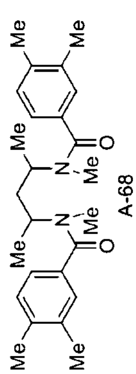
A-68

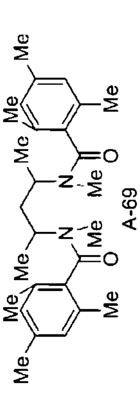
A-69

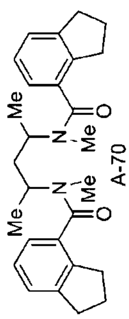
A-70

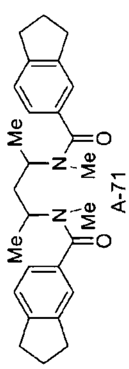
A-71

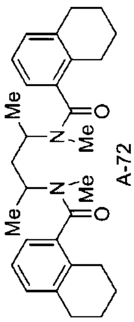
A-72

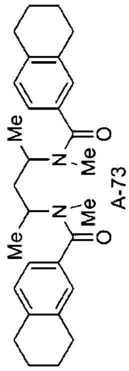
A-73

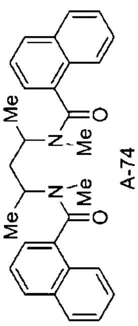
A-74

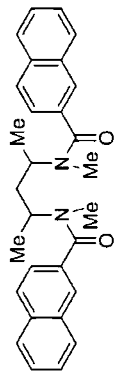
A-75

[Chem. 6]

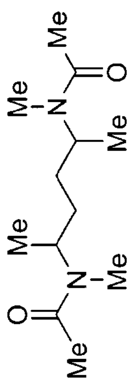
B-1

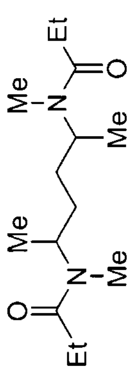
B-2

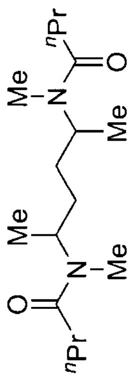
B-3

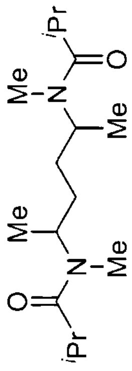
B-4

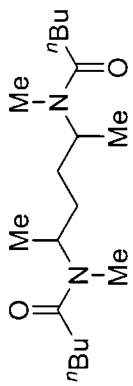
B-5

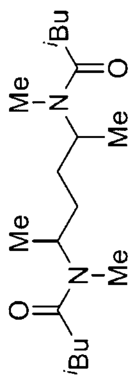
B-6

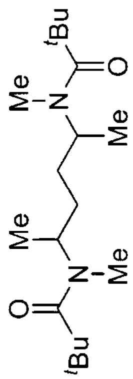
B-7

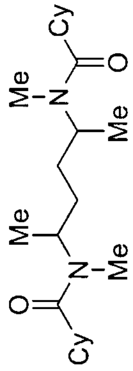
B-8

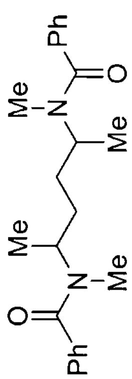
B-9

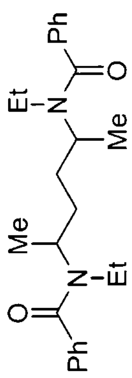
B-10

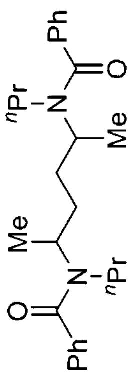
B-11

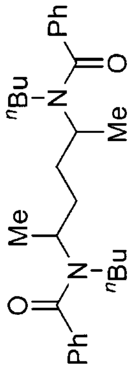
B-12

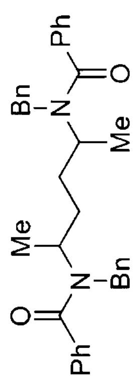
B-13

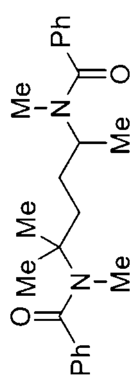
B-14

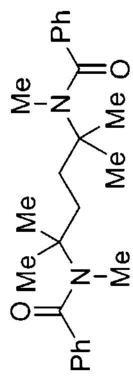
B-15

[Chem. 7]

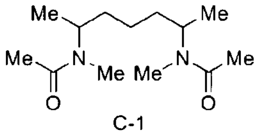
C-1

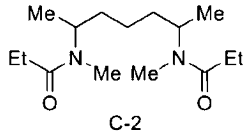
C-2

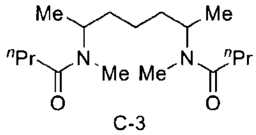
C-3

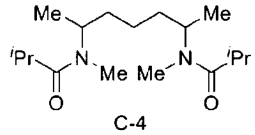
C-4

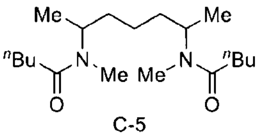
C-5

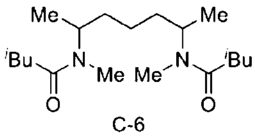
C-6

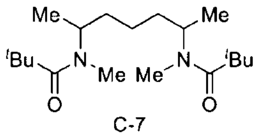
C-7

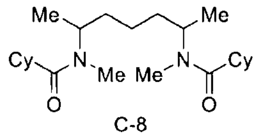
C-8

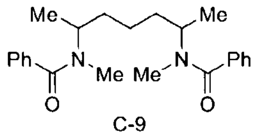
C-9

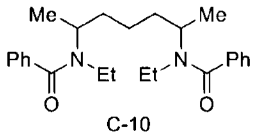
C-10

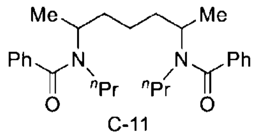
C-11

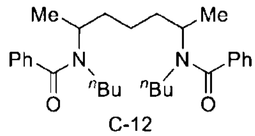
C-12

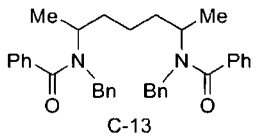
C-13

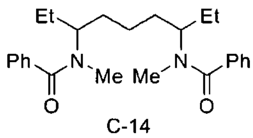
C-14

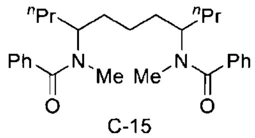
C-15

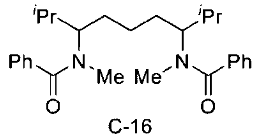
C-16

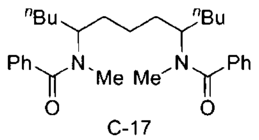
C-17

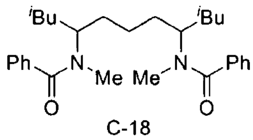
C-18

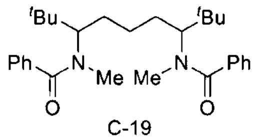
C-19

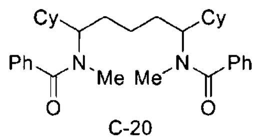
C-20

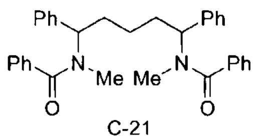
C-21

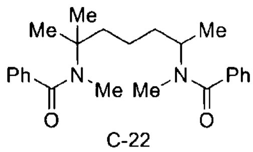
C-22

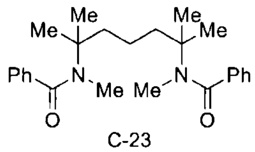
C-23

[Chem. 8]

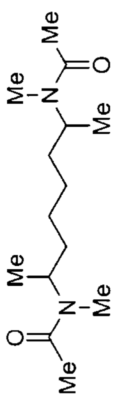
D-1

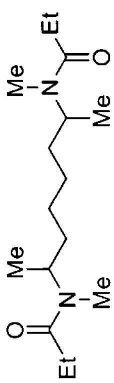
D-2

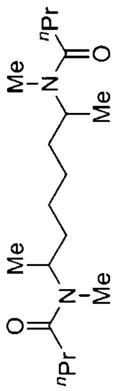
D-3

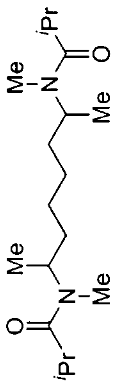
D-4

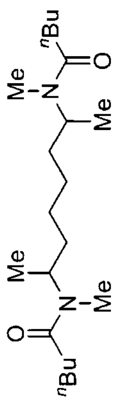
D-5

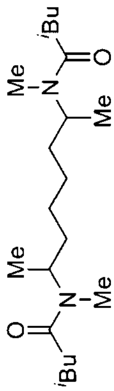
D-6

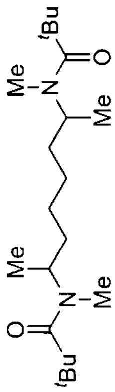
D-7

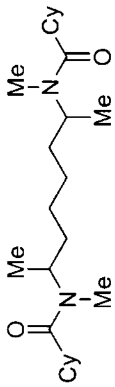
D-8

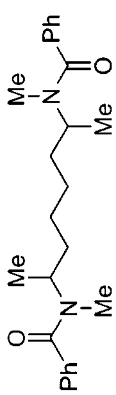
D-9

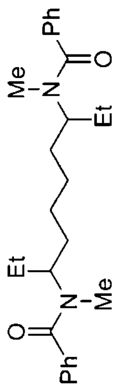
D-10

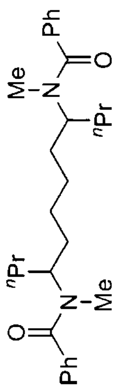
D-11

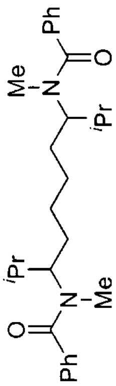
D-12

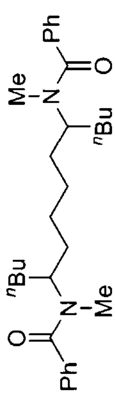
D-13

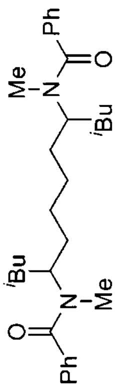
D-14

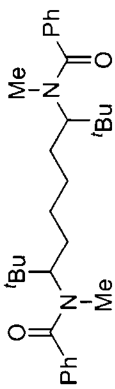
D-15

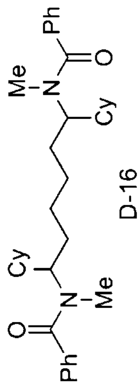
D-16

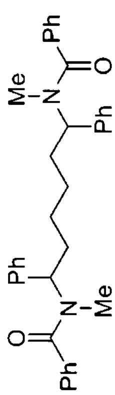
D-17

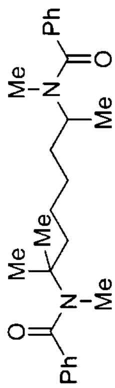
D-18

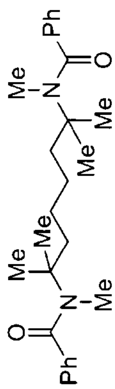
D-19

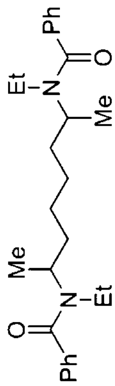
D-20

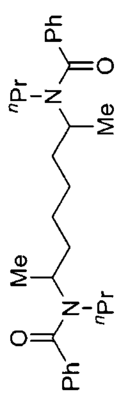
D-21

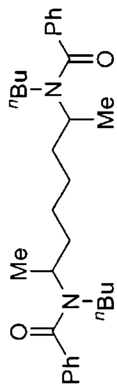
D-22

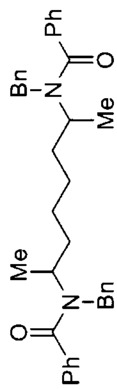
D-23

[Chem. 9]

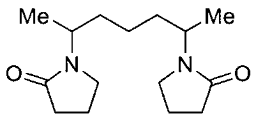
H-25

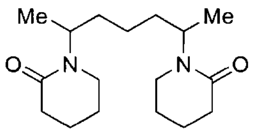
H-26

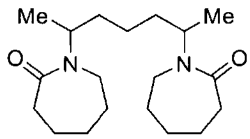
H-27

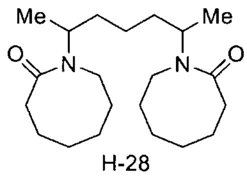
H-28

Aside from the compounds shown above, compounds having a "N-H" type amide structure to be disclosed in Examples below can also be exemplified.

In the structural formulae depicted above, a methyl group is denoted as "Me," an ethyl group is denoted as "Et," a propyl group is denoted as "Pr," a butyl group is denoted as "Bu," a phenyl group is denoted as "Ph," cyclohexyl group is denoted as "Cy," and a benzyl group is denoted as "Bn." "n" represents "normal," "i" represents "iso," and "t" represents "tertiary."

In the structural formulae, a carbon atom is present at the top or end portion thereof, and "-" is a covalent bond. The form of such compound structural formulae is a form well known to a person skilled in the art.

Such compounds can be used in a variety of applications, and in particular, an application as an internal donor of a solid titanium catalyst component that is used in a catalyst for olefin polymerization is suitable. In the case of being used as the internal donor, these compounds may be used singly, or two or more thereof may be used in combination. As long as the object of the present invention is not impaired, this amide compound can be jointly used with a known internal donor component such as a different known diamide compound or ester compound. The amide compound can also be formed in a process of preparing the solid titanium catalyst component.

When polymerization of an $\alpha$-olefin, such as propylene, is performed in the presence of an olefin polymerization catalyst for which the above-described solid titanium catalyst component is used, there is a tendency that a polymer having a broad molecular weight distribution, and a high melting point and heat of fusion in a highly active manner. Although the reason for this is not known at present, the present inventors believe that, including the contents described above, the amide moiety in the amide compound of the present invention tends to be coordinated relatively firmly with titanium and magnesium in the solid titanium catalyst component, while especially from the viewpoint of rigidity centered around the $C^1$ carbon and $C^2$ carbon that are bonded to nitrogen of the amide groups in the structural formula (1), the amide compound of the present invention is considered to have a loose structure, thus taking conformation with movement in a limited range, and therefore, an olefin polymer with high stereoregularity and broad molecular weight distribution (with a spread on the high molecular weight side) is easily provided. In that case, the stereoregularity of polymers, especially those on the high molecular weight side, is high, making them easily crystallized in spite of their molecular weight, and since the nucleating agent effect is also expressed, it is assumed that a polymer with a high melting point and heat of fusion is easily obtained.

The olefin polymer obtained by the method of the present invention is a polymer obtained using a catalyst that can take conformation with a certain degree of spread, and can thus be a polymer with a spread on the low molecular weight side as well. This may result in more components dissolving in a hydrocarbon solvent such as decane. Such dissolving components are usually feared to weaken the crystalline structure of olefin polymers, but the heat of fusion of the olefin polymer of the present invention tends to be high. This is probably because the nucleating agent effect of the high molecular weight components described above takes precedence.

<Method for producing amide compound>

A method for producing the amide compound is not particularly limited, and it is possible to use, for example, "synthesis example" in Examples to be described below. The amide compound may also be synthesized using a known reaction. The amide compound may also be synthesized by synthesizing each moiety by a known synthesis method and combining those by a known method. More specifically, the amide compound can be produced using a reaction as described below.

A diamide compound of the following reaction formula (2) can be synthesized by a reaction between an N,N'-dialkyldiamine compound and an acid chloride in the presence of, for example, a base. The diamine compound being used may be a corresponding hydrochloride. Examples of the base used can include, but not limited to, sodium hydroxide, potassium hydroxide, pyridine, N,N-dimethyl-4-aminopyridine (DMAP), and triethylamine.

[Chem. 10]

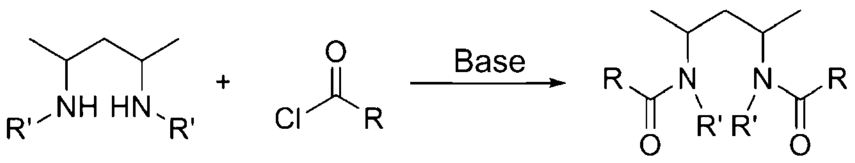


(2)

The diamide compound shown in the reaction formula (2) can also be synthesized by the method including reacting an N,N'-dialkyldiamine compound with carboxylic acid in the presence of an acid catalyst or the method using a condensing reagent, such as N,N'-dicyclohexylcarbodiimide (DCC) as shown in the following reaction formula (3).

[Chem. 11]

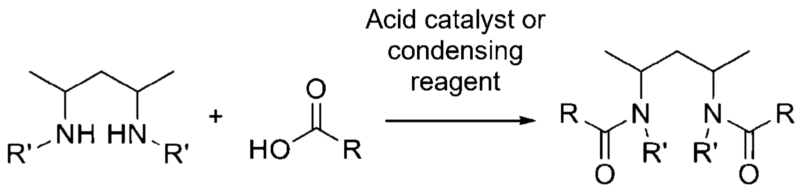


(3)

Furthermore, the diamide compound shown in the reaction formula (3) can also be synthesized by reacting a diamide compound and a base, which correspond to each other, as shown in the following reaction formula (4) and then reacting the reaction product with an alkyl halide. The base being used is not particularly limited, and examples thereof include organolithium reagents, sodium hydride, and potassium hydride.

[Chem. 12]

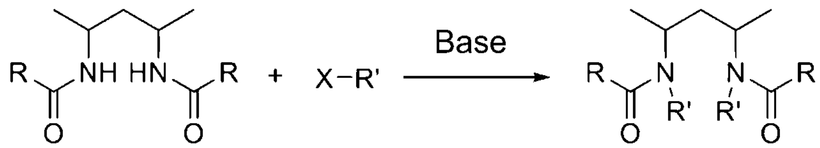


(4)

As described above, the amide compound of the present invention can be used in a variety of applications. Particularly, the amide compound is suitable as an internal donor component of a solid titanium catalyst component that is contained in a catalyst for olefin polymerization as described above. The amide compound can also be expected to be used as, aside from the above-described applications, a special solvent, an intermediate of a medicine or pesticide, and a raw material of a special polyamide.

Examples

(Method for analyzing compounds)

A $^{1}H$-NMR spectrum (400 MHz, manufactured by JEOL Ltd., JNM-ECZ400S/L1 type measuring instrument) was measured, the peaks were assigned by a routine method to determine a structure.

[Example 1]

<Synthesis of compound 1>

A compound 1 shown below was synthesized by a method described below.

[Chem. 13]

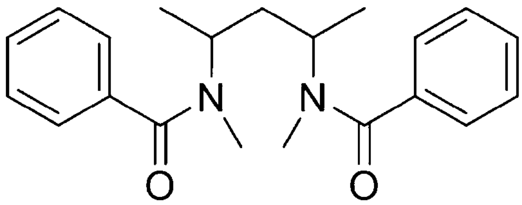

(Compound 1)

5.05 g of N,N'-dimethyl-2,4-pentanediamine (38.8 mmol, 1 eq) and 39 mL of pyridine (dehydrated) were added into an oven dried 100 mL three-necked flask containing a magnetic stirring bar in a nitrogen atmosphere. After the reaction solution was cooled in an ice bath, 11.7 g of benzoyl chloride (83.0 mmol, 2.1 eq) was slowly added dropwise thereto. After the end of the dropwise addition, the reaction temperature was slowly raised to room temperature, and the reaction solution was continuously stirred for 22 hours. After the end of the reaction, the reaction solution was cooled again in an ice bath, and 5 mL of methanol was added thereto. Water and ethyl acetate were added to the generated reaction solution, and the resulting aqueous layer was extracted with ethyl acetate three times. The combined organic layers were washed once with each of water and brine, dried over magnesium sulfate, and then concentrated with a rotary evaporator. The obtained crude product was purified by NH gel column chromatography (hexane:ethyl acetate = gradient from 90:10 to 40:60) to obtain 11.7 g of the compound 1 (35.1 mmol, yield 90%). The $^1$H-NMR data of the obtained compound 1 are shown below.

$^1$H NMR (400 MHz, $CDCl_3$, TMS as internal standard): $\delta$ 0.94-1.32 (m, 6H), 1.57-1.90 (m, 2 H, overlapped with the signal of $H_2O$), 2.37-3.00 (m, 6H), 3.63-4.96 (m, 2H), 6.87-7.46 (m, 10 H, overlapped with the signal of residual $CHCl_3$).

<Preparation of solid titanium catalyst component [α1]>

After a 1-liter glass vessel was sufficiently purged with nitrogen, 85.8 g of anhydrous magnesium chloride, 321 g of decane, and 352 g of 2-ethylhexyl alcohol were placed therein, and thermally reacted at 130°C for 3 hours to give a homogeneous solution. Then, 241 g of this solution and 6.43 g of ethyl benzoate were added to the glass vessel, and mixed by being stirred at 50°C for 1 hour.

After the homogeneous solution thus obtained was cooled to room temperature, the entirety of 38.3 ml of the homogeneous solution was added dropwise over 45 min to 100 ml of titanium tetrachloride retained at -20°C while being stirred. After the addition was finished, the temperature of this mixture was raised to 80°C over 3.8 hours. When the temperature reached 80°C, 1.71 g of the compound 1 was added into the mixture. The temperature was again raised to 120°C over 40 min, and the mixture was retained at the same temperature for 35 min while being stirred. After the reaction was complete, the solid portion was recovered by hot filtration, resuspended in 100 ml of titanium tetrachloride, and again thermally reacted at 120°C for 35 min while being stirred. After the reaction was complete, the solid portion was recovered again by hot filtration and thoroughly washed with decane at 100°C and decane at room temperature until no free titanium compound was detected in the washing liquid. The solid titanium catalyst component [α1] prepared by the above operations was preserved as a decane slurry, and a part of the slurry was dried to check the catalyst composition. The composition of the solid titanium catalyst component [α1] thus obtained included 0.66% by mass of titanium, 1.3% by mass of magnesium, and 0.05% by mass of 2-ethylhexyl alcohol residues.

<Main polymerization>

After the addition of 500 g of propylene and 1 NL of hydrogen at room temperature to a polymerization vessel having an inner capacity of 2 L, a mixture obtained by mixing 7 mL of heptane, 0.50 mmol of triethyl aluminum, 0.10 mmol of cyclohexylmethyldimethoxysilane, and 0.0040 mmol of the solid titanium catalyst component [α1] (in terms of titanium atom) at 25°C for 10 minutes was added thereto, and the temperature inside the polymerization vessel was rapidly raised up to 70°C under stirring. After polymerization at 70°C for 1.5 hours, the reaction was stopped with a small amount of ethanol, and propylene was purged. Furthermore, obtained polymer particles were dried under reduced pressure at 80°C overnight. A variety of polymerization results are as described below.

Activity: 13.4 kg-PP/g-catalyst
Bulk specific gravity: 380 Kg/$m^3$
MFR: 10.1 g/10 min
Decane-insoluble part content: 9.0% by mass

Tm: 163.0°C, 157.6°C
Tc: 112.6°C
Tmf: 169.0°C
ΔH: 89.7 J/g
Mw/Mn: 9.1
Mz/Mw: 8.8

A variety of analysis methods are as described below.

(1) Bulk specific gravity:

The bulk specific gravity was measured in accordance with JIS K-6721.

(2) Melt flow rate (MFR):

In accordance with ASTM D1238E, the measurement temperature for a propylene polymer was set to 230°C with a load of 2.16 kg.

(3) Amount of decane-soluble (insoluble) part:

A glass measuring container was charged with about 3 g of the propylene polymer (measured up to an accuracy of $10^{-4}$ g; the weight indicated by b (gram) in the following formula), 500 ml of decane, and a small amount of a heat-resistant stabilizer soluble in decane, and the propylene polymer was dissolved by being heated to 150°C over two hours while being stirred with a stirrer in a nitrogen atmosphere, allowed to stand at 150°C for two hours, and gradually cooled to 23°C over eight hours. A liquid containing an obtained precipitate of the propylene polymer was filtered under reduced pressure with a 25G-4 standard glass filter manufactured by Tokyo Garasu Kikai Co., Ltd. 100 ml of a filtrate was recovered and dried under reduced pressure to obtain a part of a decane-soluble part, and this weight was measured up to an accuracy of $10^{-4}$ g (this weight was indicated by a (g) in the following formula). After this operation, the amount of the decane-soluble part was determined by the following formula.

$$\text{Decane-soluble part content} = 100 \times (500 \times a)/(100 \times b)$$

$$\text{Decane-insoluble part content} = 100 - 100 \times (500 \times a)/(100 \times b)$$

(4) Molecular weight distribution (MWD):

Gel permeation chromatograph: HLC-8321 GPC/HT type manufactured by Tosoh Corporation
Detector: Differential refractometer
Column: Two TSKgel GMH6-HTs and two TSKgel GMH6-HTLs manufactured by Tosoh Corporation were serially connected.
Mobile phase medium: o-Dichlorobenzene
Flow rate: 1.0 ml/minute
Measurement temperature: 140°C
Method of creating calibration curve: A standard polystyrene sample was used.
Sample concentration: 0.1% (w/w)
Amount of sample solution: 0.4 ml

Measurement was performed under the above-described conditions, an obtained chromatogram was analyzed by a known method to calculate the weight average molecular weight (Mw), the number average molecular weight (Mn), the Z average molecular weight (Mz), the Mw/Mn value and the Mz/Mw value, which are indices of the molecular weight distribution (MWD). The measurement time per sample was 60 minutes.

(5) Melting point (Tm) of polymer:

The melting point (Tm), crystallization temperature (Tc), and amount of heat of fusion (ΔH) of the polymer in the present invention were measured with a differential scanning calorimeter (DSC) that was a DSC 8000 device manufactured by PerkinElmer Co., Ltd. 3 to 10 mg of a specimen was sealed in an aluminum pan and heated from room temperature to 200°C at 100°C/minute. This specimen was retained at 200°C for five minutes and then cooled to 30°C at 10°C/minute. A peak temperature that was observed in this cooling test was regarded as the crystallization temperature (Tc), and the amount of heat generated that was specified by the area of the peak was regarded as ΔH. Subsequently, the specimen was retained at 30°C for five minutes and then heated for the second time to 200°C at 10°C/minute. A peak temperature that was observed by this second heating test was regarded as the melting point (Tm).

The final melting point (Tmf) of the polymer in the present invention was measured with a differential scanning calorimeter (DSC) that was the DSC 8000 device manufactured by PerkinElmer Co., Ltd. 3 to 10 mg of a specimen was sealed in an aluminum pan and heated from room temperature to 240°C at 80°C/minute. The specimen was retained at 240°C for one minute and then cooled to 0°C at 80°C/minute. The specimen was retained at 0°C for one minute, then heated to 150°C at 80°C/minute, and retained for five minutes. Finally, the specimen was heated to 180°C at 1.35°C/minute, and the intersection between the tangent of the inflection point on the high-temperature side of a peak that was obtained in this final heating test and the base line was employed as the final melting point (Tmf).

Tmf can be considered as one of the parameters for evaluating, for example, the crystal structure of a component exhibiting extremely high stereoregularity or the ease of crystallization or crystal structure of a polymer in an ultra-high molecular weight region, which is considered to have a tendency of being less likely to crystallize. More specifically, it can be considered that as the value of this Tmf is higher, an ultra-high molecular weight polymer component is more likely to form highly heat-resistant crystals.

[Example 2]

<Synthesis of compound 2>

A compound 2 shown below was synthesized by a method described below.

[Chem. 14]

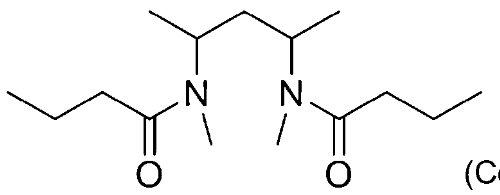

(Compound 2)

4.90 g (18.1 mmol, yield 79%) of the compound 2 was obtained in accordance with the operation and equivalence relationship described in <Synthesis of compound 1> except that butyryl chloride was used in place of using benzoyl chloride in <Synthesis of compound 1> as described above. The $^1$H-NMR data of the obtained compound 2 are shown below.

$^1$H NMR (400MHz, $CDCl_3$, TMS as internal standard): 0.91-1.00 (m, 6H), 1.04-1.28 (m, 6H), 1.38-1.83 (m, 6H, overlapped with the signal of $H_2O$), 2.05-2.35 (m, 4H), 2.75-2.84 (m, 6H), 3.66-4.85 (m, 2H).

<Preparation of solid titanium catalyst component [α2]>

A solid titanium catalyst component [α2] was obtained in the same manner as Example 1, except that 1.37 g of the compound 2 was used in place of 1.77 g of the compound 1. The composition of the solid titanium catalyst component [α2] thus obtained was 0.64% by mass of titanium, 1.4% by mass of magnesium, and 0.07% by mass of a 2-ethylhexyl alcohol residue.

<Main polymerization>

Polymerization of propylene was performed in the same manner as Example 1, except that the solid titanium catalyst component [α2] was used in place of the solid titanium catalyst component [α1]. A variety of polymerization results are as described below.

Activity: 14.6 kg-PP/g-catalyst
Bulk specific gravity: 470 $Kg/m^3$
MFR: 13.0 g/10 min
Decane-insoluble part content: 8.0% by mass
Tm: 158.7°C
Tc: 114.4°C
Tmf: 169.2°C
$\Delta$H: 90.3 J/g
Mw/Mn: 8.8
Mz/Mw: 8.3

[Example 3]

<Synthesis of compound 3>

A compound 3 shown below was synthesized by a method described below.

[Chem. 15]

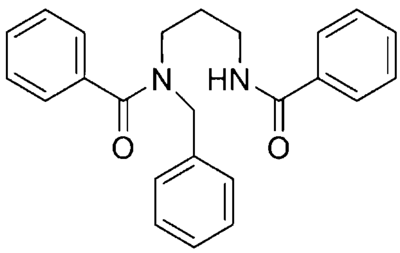

(Compound 3)

An oven dried 100 mL three-necked flask containing a magnetic stirring bar was prepared, and the internal atmosphere was replaced with nitrogen. 0.99 g of N-benzyl-1,3-propanediamine (12.1 mmol, 1 eq) and 24 mL of pyridine (dehydrated) were added thereto in the same nitrogen atmosphere, and the reaction solution was cooled in an ice bath. After it was confirmed that the reaction solution had been sufficiently cooled, 3.56 g of benzoyl chloride (25.3 mmol, 2.1 eq) was slowly added dropwise thereto. After the end of the dropwise addition, the reaction temperature was slowly raised to room temperature, and the reaction solution was continuously stirred for 20 hours. After reaction monitoring by GC-MS analysis, the reaction solution was cooled again in an ice bath and quenched by adding 2 mL of methanol thereto. Ethyl acetate and water were added to the quenched reaction solution, and the reaction solution was extracted with ethyl acetate three times. The combined organic layers were washed once with each of water, a saturated sodium bicarbonate aqueous solution, and brine, and the obtained organic layer was dried over magnesium sulfate and then concentrated with a rotary evaporator. The obtained crude product was purified by NH gel column chromatography (hexane:ethyl acetate = gradient from 100:0 to 60:40) to obtain 4.34 g of TKN-040(compound 3) (11.7 mmol, yield 97%). The product was highly viscous, and a small amount of the ethyl acetate remained. The $^1$H-NMR data of the obtained compound 3 are shown below.

$^1$H-NMR (500 MHz, $CDCl_3$, TMS as internal standard): $\delta$ 1.80-1.85 (m, 2H), 3.51-3.55 (m, 2H), 3.65-3.68 (m, 2H), 4.54 (s, 2H), 7.17-7.19 (m, 2H), 7.29-7.49 (m, 10H), 7.87 (br s, 1H), 7.94-7.96 (m, 2H).

## Claims

1. An amide compound having a structure of the following formula (1):

[Chem. 1]

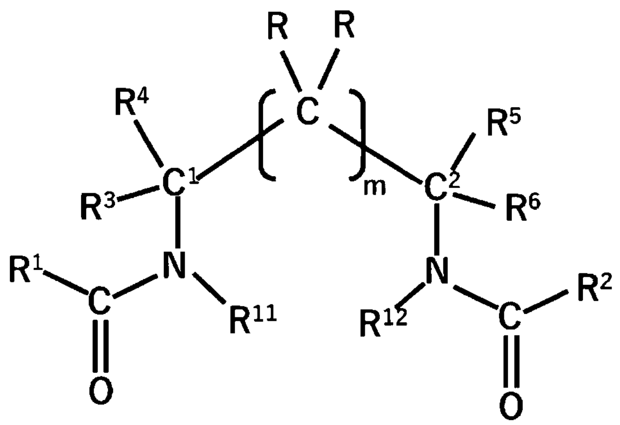

(1)

wherein

C, $C^1$, and $C^2$ are carbon atoms,
N is a nitrogen atom,
$R^1$ and $R^2$ are each a hydrocarbon group,
$R^{11}$ and $R^{12}$ are each a hydrogen atom or a hydrocarbon group, and at least one group thereof is a hydrocarbon group,
$R^3$ to $R^6$ are each a hydrogen atom or a hydrocarbon group,
R is a hydrogen atom, and
m is an integer of 1 to 5.

2. The amide compound according to Claim 1, wherein both the $R^{11}$ and $R^{12}$ are hydrocarbon groups.

3. The amide compound according to Claim 1, wherein the $R^1$, $R^2$, $R^4$, and $R^5$ are hydrocarbon groups composed of carbon and hydrogen.

## INTERNATIONAL SEARCH REPORT

International application No.
**PCT/JP2024/013474**

**A. CLASSIFICATION OF SUBJECT MATTER**

***C07C 233/36***(2006.01)i; ***C07C 233/78***(2006.01)i
FI: C07C233/36 CSP; C07C233/78

According to International Patent Classification (IPC) or to both national classification and IPC

**B. FIELDS SEARCHED**

Minimum documentation searched (classification system followed by classification symbols)

C07C233/36; C07C233/78

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | HESSE, K.-D., Synthese von Pyrazolidinen, Justus Liebigs Annalen der Chemie, 1971, vol. 743, no. 1, pp. 50-56, DOI:10.1002/jlac.19717430106<br>p. 52 | 1-3 |
| X | WO 2018/226732 A1 (FLAGSHIP PIONEERING INNOVATIONS V, INC.) 13 December 2018 (2018-12-13)<br>p. 135 | 1-2 |
| X | WO 2015/116968 A1 (CASE WESTERN RESERVE UNIVERSITY) 06 August 2015 (2015-08-06)<br>scheme 1 | 1-2 |
| X | ZHANG, Z. et al., Selective Monoacylation of Symmetrical Diamines via Prior Complexation with Boron, Organic Letters, 2003, vol. 5, no. 19, pp. 3399-3402, DOI: 10.1021/ol0300773<br>table 3 | 1-2 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 June 2024** | **18 June 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

INTERNATIONAL SEARCH REPORT

International application No.
PCT/JP2024/013474

C. DOCUMENTS CONSIDERED TO BE RELEVANT

| Category* | Citation of document, with indication, where appropriate, of the relevant passages | Relevant to claim No. |
|---|---|---|
| X | CIRRINCIONE, G. et al., Rotational isomerism of NN'-dimethyl-α,ω-bis(benzoy lamino)alkanes, J. Chem. Soc., Perkin Trans. 2, 1984, no. 6, pp. 1089-1091, DOI:10.1039/P 29840001089<br>p. 1089 | 1-2 |
| X | VEITH, H. J. et al., Neuartige massenspektrometrische Zerfallsreaktionen bei α,ω-disubstit uierten Alkanen. 15. Mitteilung uber das massenspektrometrische verhalten von stickstoffve rbindungen [1], Helvetica Chimica Acta, 1971, vol. 54, no. 2, pp. 653-680, DOI:10.1002/hlc a.19710540224<br>table 3 | 1-2 |
| X | BILLMAN, J. H. et al., Synthesis of Disecondary Amines; N,N'-Diphenyl-α, ω-Diaminoalkanes, The Journal of Organic Chemistry, 1951, vol. 16, no. 7, pp. 1041-1046, DOI: 10.1021/jo50001a004<br>tables 1, 4 | 1-2 |
| X | Database Registry, 2015, RN 1824403-71-5, retrieved from STN international [online]: retrieved on 3 June 2024 | 1, 3 |
| X | WO 2010/086366 A1 (NOVARTIS AG) 05 August 2010 (2010-08-05)<br>pp. 52, 101 | 1 |
| X | US 5421868 A (INTERNATIONAL BUSINESS MACHINES CORPORATION) 06 June 1995 (1995-06-06)<br>column 5 | 1 |
| X | SCHOPP, E. et al., Beitrag zur Massenspektrometrie substituierter α, ω-Alkandiamine. 29. Mitteilung uber massenspektrometrische Untersuchungen, Helvetica Chimica Acta, 1977, vol. 60, no. 7, pp. 2425-2430, DOI:10.1002/hlca.19770600729<br>p. 2426 | 1 |
| A | CN 108570119 A (BEIJING LIHE ZHIXIN TECHNOLOGY CO., LTD.) 25 September 2018 (2018-09-25)<br>entire text | 1-3 |
| A | CN 108570120 A (BEIJING LIHE ZHIXIN TECHNOLOGY CO., LTD.) 25 September 2018 (2018-09-25)<br>entire text | 1-3 |
| A | WO 2011/106500 A1 (DOW GLOBAL TECHNOLOGIES LLC) 01 September 2011 (2011-09-01)<br>entire text | 1-3 |

Form PCT/ISA/210 (second sheet) (July 2022)

**INTERNATIONAL SEARCH REPORT**
**Information on patent family members**

International application No.
**PCT/JP2024/013474**

| Patent document cited in search report | Publication date (day/month/year) | Patent family member(s) | Publication date (day/month/year) |
|---|---|---|---|
| WO 2018/226732 A1 | 13 December 2018 | JP 2020-522514 A<br>US 2020/0101030 A1<br>EP 3634430 A1<br>KR 10-2020-0011972 A<br>CN 110869024 A | |
| WO 2015/116968 A1 | 06 August 2015 | US 2016/0346236 A1 | |
| WO 2010/086366 A1 | 05 August 2010 | US 2010/0222396 A1 | |
| US 5421868 A | 06 June 1995 | (Family: none) | |
| CN 108570119 A | 25 September 2018 | (Family: none) | |
| CN 108570120 A | 25 September 2018 | (Family: none) | |
| WO 2011/106500 A1 | 01 September 2011 | JP 2013-521341 A<br>US 2012/0316299 A1<br>EP 2539379 A1<br>CN 102918068 A<br>KR 10-2013-0020771 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005226076 A
- JP 2000516987 A
- JP 2002542347 A
- JP 2005517746 A
- JP 2011529888 A
- JP 2014500390 A
- CN 108570120
- CN 108570119